# FASCICULE DE BREVET EUROPEEN

(11) **EP 0 877 584 B1**
(45) Date de publication et mention de la délivrance du brevet: **01.10.2003**
(21) Numéro de dépôt: 97902402.3
(22) Date de dépôt: 30.01.1997
(51) Int. Cl.: A61F 2/14

(54) **DISPOSITIF DE KERATOPROTHESE**
KERATOPLASTIK-VORRICHTUNG
CORNEAL PROSTHESIS DEVICE

(30) Priorité: 30.01.1996 FR 9601066
(43) Date de publication de la demande: 18.11.1998
(73) Titulaire: CORNEAL INDUSTRIE, 74370 Pringy (FR)
(72) Inventeur: LACOMBE, Emmanuel, F-92200 Neuilly (FR); BOS, Gilles, F-74330 La Balme-de-Silingy (FR); VILLAIN, Franck, F-74000 Annecy (FR)
(74) Mandataire: Dronne, Guy
(86) Numéro de dépôt international: FR9700178
(87) Numéro de publication internationale: WO97027824

(56) Documents cités:
- EP-A- 0 469 993
- WO-A-89/00032
- WO-A-93/13731
- FR-A- 2 687 564
- US-A- 2 952 023
- US-A- 4 842 599
- US-A- 5 300 115
- DATABASE WPI Section PQ, Derwent Publications Ltd., London, GB; Class P, AN 93-099320 XP002012131 & SU 1 727 823 A (KABANOV I.B.)
- DATABASE WPI Section PQ, Derwent Publications Ltd., London, GB; Class P, AN 93-157590 XP002012132 & SU 1 734 725 A (ARKHAN MED INST)

## Description

La présente invention a pour objet un dispositif de kératoprothèse.

De façon plus précise, l'invention se rapporte à une prothèse optique qui peut être mise en place par la réalisation d'une incision centrale pratiquée dans la cornée de l'oeil lorsque celle-ci ne peut plus remplir sa fonction de transparence. De telles prothèses optiques sont également appelées parfois cornées artificielles.

Certaines cécités peuvent être provoquées par une opacification ou altération des propriétés optiques de la cornée. Dans certains cas, il est possible d'y remédier par kératoplastie, c'est-à-dire en remplaçant la partie centrale malade de la cornée par un fragment de cornée saine et transparente d'un donneur. Cependant, si les lésions dont est atteinte la cornée sont trop importantes (brûlure, syndrome sec, pseudo-pemphigus), il faut avoir recours à la chirurgie palliative qui consiste à ménager un orifice ou trépanation dans la partie malade de la cornée et à mettre en place une prothèse formant cornée artificielle, cette technique est dite kératoprothèse.

Le document EP-0 469 993 décrit un dispositif de kératoprothèse selon le preambule de la revendication 1.

Cette disposition permet effectivement une mise en place aisée et efficace de la kératoprothèse et son maintien dans la cornée. Cependant, il s'avère que, entre la périphérie de la prothèse et le bord de l'incision réalisée dans la cornée, il n'y a pas de véritable adhérence, ce qui ouvre une voie de passage pour les microbes vers l'intérieur de l'oeil. On comprend que cette situation peut être très dommageable pour certaines interventions.

Un objet de la présente invention est de fournir un dispositif de kératoprothèse perfectionné qui permette une mise en place aisée, tout en assurant une très bonne étanchéité entre la partie optique de la kératoprothèse et la périphérie de la trépanation réalisée dans la partie centrale de la cornée.

Pour atteindre ce but, selon l'invention, le dispositif de kératoprothèse destiné à être mis en place dans la cornée comprend les caractéristiques de la revendication 1.

On comprend que, grâce à la présence de la jupe antérieure annulaire réalisée en un matériau bio-colonisable, il est possible de provoquer par différentes techniques une colonisation effective de cette jupe antérieure de manière à réaliser une solution de continuité étanche entre la face antérieure de la cornée et la partie optique de la kératoprothèse. On comprend également que la présence de la jupe postérieure appliquée contre la face postérieure ou interne de la cornée permet d'éviter les risques d'expulsion de la kératoprothèse sous l'effet de la pression intraoculaire.

Selon un premier mode de mise en oeuvre, la partie optique et la jupe postérieure sont réalisées en PMMA. Selon une variante de réalisation, cette même partie optique est réalisée en un matériau optique souple qui peut être un gel de silicone ou un acrylique hydrophile ou non hydrophile.

Selon un mode préféré de mise en oeuvre, le matériau bio-colonisable est choisi parmi ceux qui sont couramment utilisés pour la chirurgie réparatrice et notamment le polytétrafluoroéthylène et le polyéthylène micro poreux.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées sur lesquelles:
- La figure 1 est une vue en coupe diamétrale d'un premier mode de réalisation de la kératoprothèse;
- La figure 2 est une en coupe selon le même plan d'un deuxième mode de réalisation de la kératoprothèse;
- La figure 3 est une vue en coupe diamétrale d'un troisième mode de réalisation de la kératoprothèse; et
- Les figures 4a et 4b illustrent les étapes d'un mode préféré de fabrication de la kératoprothèse selon l'invention.

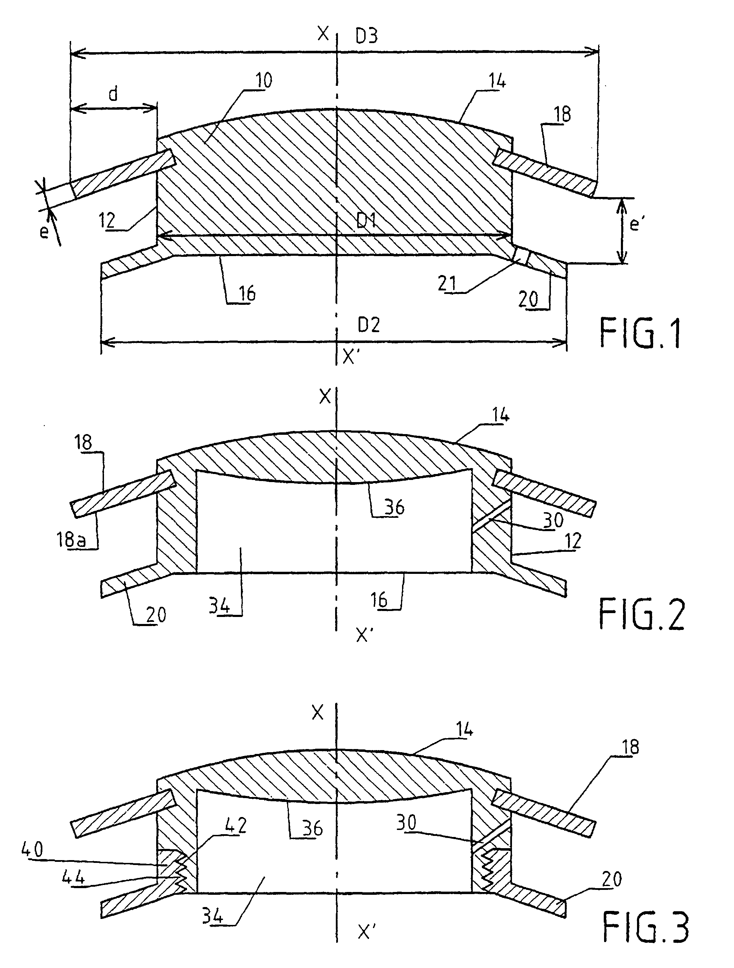

En se référant tout d'abord à la figure 1, on va décrire un premier mode de réalisation du dispositif de kératoprothèse. Ce dispositif est constitué par une partie optique 10 qui a une forme générale cylindrique de révolution autour de l'axe optique XX'. La partie optique comporte ainsi une paroi latérale 12 sensiblement cylindrique, une face avant 14 définissant un dioptre optique pour la correction de la vision et une extrémité arrière 16 qui, dans ce mode de réalisation, constitue un deuxième dioptre optique. La kératoprothèse comporte également une jupe antérieure rapportée 18 solidaire de la paroi latérale 12 de la partie optique à proximité du dioptre optique 14. Cette jupe antérieure présente un diamètre D3 supérieur au diamètre D1 de la partie optique. Enfin, la kératoprothèse comporte une jupe postérieure 20 qui fait saillie hors de la paroi latérale 12 de la partie optique à son extrémité postérieure 16. Cette jupe peut de préférence avoir une forme légèrement tronconique en retrait par rapport à la face postérieure 16. Son diamètre est égal à D2. De préférence, la jupe antérieure 18 présente la même conicité que la jupe postérieure de telle manière que les deux jupes soient sensiblement parallèles.

Selon un premier mode de réalisation de la kératoprothèse, la partie optique 10 est réalisée en un matériau transparent rigide tel que du PMMA. Pour participer au maintien de la jupe antérieure 18, la face antérieure 14 de la partie optique peut être prolongée par un rebord annulaire 22 qui recouvre partiellement la face antérieure de la jupe antérieure. Selon une caractéristique essentielle de l'invention, la jupe antérieure 18 est constituée par un polymère de synthèse bio-compatible et bio-colonisable. Il s'agit donc d'un matériau micro poreux. De préférence, le matériau bio-colonisable 18 consiste en du polytetrafluorocthylène ou un polyéthylène. De préférence encore, le matériau constituant la jupe antérieure 18 présente des pores dont le diamètre est compris entre 20 et 100 microns. La porosité du matériau, c'est-à-dire le vide est de préférence supérieure à 50% du volume total. Pour permettre une colonisation efficace du matériau par le tissu vivant, il faut que les "cavités" résultant de la porosité du matériau soient ouvertes, c'est-à-dire communiquent entre elles pour réaliser des "chemins" de colonisation traversant la jupe d'une face à l'autre afin qu'il y ait une colonisation suffisante et que cette colonisation par les tissus reste "vivante". Par exemple, un tel matériau peut être obtenu par frittage de microbilles de diamètres différents. C'est ce sens qu'il faut donner à l'expression "matériau microporeux".

Le diamètre D1 de la partie optique qui procure la correction optique présente donc un diamètre compris entre 3 et 7 mm, de préférence égal à 5mm. La jupe antérieure 18 présente une épaisseur e qui est de préférence comprise entre 20 et 500 microns, afin de présenter une résistance mécanique suffisante tout en procurant des conditions de colonisation optimales. De préférence encore, cette épaisseur est comprise entre 50 et 200 microns. La distance e' entre la face postérieure de la jupe antérieure 18 et la face antérieure de la jupe postérieure 20 est comprise de préférence entre 400 et 700 microns, ce qui correspond à l'épaisseur d'une cornée saine normale. Plus généralement, pour s'adapter à différentes épaisseurs possibles de cornée, éventuellement atteintes de pathologie, la longueur e' peut être comprise entre 300 microns et 2 mm. En ce qui concerne la jupe postérieure 20, son épaisseur peut être comprise entre 100 et 500 microns et typiquement être égale à 250 microns. Pour assurer une bonne tenue mécanique de la kératoprothèse, la longueur d dont la jupe antérieure dépasse de la paroi latérale de la partie optique est comprise de préférence entre 1 et 2 mm, ce qui conduit à un diamètre D3 compris entre 5 et 11mm. De préférence, la jupe postérieure 20 présente des trous 21 pour réaliser un suturage de la kératoprothèse sur la cornée. Le diamètre D2 peut être légèrement inférieur à D3. Il doit cependant être suffisant pour empêcher l'expulsion de la prothèse sous l'effet de la pression oculaire et pour permettre la réalisation des trous de suturage.

Dans le cas de la kératoprothèse de la figure 2, la partie optique 10 comporte une cavité 34 sensiblement cylindrique qui débouche dans la face postérieure 16 de la kératoprothèse, le fond de la cavité 34 définissant une surface optique 36 qui peut être concave, convexe ou plane. Le système optique correcteur est donc constitué par la portion de la partie optique limitée par les dioptres 14 et 36. Une telle solution est particulièrement intéressante dans le cas où la partie optique n'est pas réalisée avec un matériau rigide tel que le PMMA mais à l'aide d'un matériau souple couramment utilisé pour les implants intraoculaires. Ce matériau souple peut être un gel de silicone ou un hydrogel, notamment un acrylique hydrophile dont un bon exemple est le pHEMA. Le fait que le matériau soit souple et que la partie optique ait une épaisseur réduite permet de mesurer la pression oculaire malgré la présence de la kératoprothèse. Il pourrait également être utilisé pour réaliser la partie optique du mode de réalisation de la figure 1.

La figure 2 montre également la possibilité de prévoir un canal de section réduite 30 traversant une partie de la partie optique et faisant communiquer la cavité 34 de la partie optique avec la paroi latérale 12 de cette partie optique à proximité de la face postérieure 18a de la jupe antérieure 18. Ce canal de très faible section permet le drainage de l'humeur aqueuse. La présence de ce canal permettra de lutter efficacement contre une élévation de pression intraoculaire associée au glaucome. Comme le canal débouche en retrait de la jupe antérieure 18, ce canal est protégé vis-à-vis d'une invasion possible par des microbes. La section réduite 30 de ce canal peut être modifiée et agrandie à l'aide d'un laser. Elle sera donc prévue obturée au départ puis ouvert à l'aide d'un laser YAG selon besoin.

Il va de soi qu'un canal semblable au canal 30 pourrait être prévu dans le mode de réalisation de la figure 1. Il ferait alors communiquer la face postérieure 16 et la paroi latérale 12.

La figure 3 montre un troisième mode de réalisation de la kératoprothèse qui se distingue du mode de réalisation de la figure 2 simplement par le fait que la jupe postérieure 20 est constituée par une pièce séparée de la partie optique 10. Pour cela, la jupe 20 peut être prolongée par un manchon 40 muni d'un taraudage 42. La partie correspondante de la paroi latérale de la partie optique est bien sûr elle-même munie d'un filetage 44. On comprend qu'ainsi, il est éventuellement possible de procéder au "démontage" de la kératoprothèse par dévissage de la partie optique par rapport à la jupe 20. Cette possibilité de démontage peut éventuellement être utilisée pour nettoyer la face postérieure 16 ou 36 de la kératoprothèse qui aurait été recouverte par une membrane opaque.

On comprend que, dans tous les modes de réalisation, la jupe antérieure rapportée, réalisée en un matériau bio-colonisable, permet d'une part une certaine solidarisation de la kératoprothèse avec la cornée et surtout, d'autre part, une étanchéité totale entre la cornée et la kératoprothèse vis-à-vis des microbes.

Si le tissu constituant une cornée saine peut coloniser le matériau microporeux, la colonisation par le tissu de la cornée pathologique est plus hypothétique. Il est préférable d'obtenir la colonisation par un apport de tissu sain de type muqueuse buccale ou conjonctive.

Il doit bien sûr exister une liaison mécanique entre la partie optique et la jupe antérieure 18. Cette liaison peut être obtenue en disposant la jupe antérieure dans un moule qui sert à fabriquer la partie optique par moulage par injection, la jupe antérieure jouant le rôle d'insert. Après démoulage, on obtient une prothèse dans laquelle la jupe antérieure est solidaire de la partie optique.

On peut également réaliser la liaison mécanique par fusion partielle localisée et temporaire du PMMA et du matériau microporeux. Cette fusion partielle peut être obtenue par ultrasons ou par dissolution chimique. Dans tous les cas, il est bien sûr nécessaire de réaliser entre ces deux pièces une liaison étanche et présentant une bonne résistance mécanique.

On comprend également que la jupe postérieure a sa face antérieure qui est appliquée contre la face postérieure ou interne de la cornée. Ainsi, même sous l'effet de la pression intraoculaire, la kératoprothèse ne risque pas d'être expulsée hors de l'oeil puisque cette pression tend à plaquer la jupe postérieure sur la face postérieure de la cornée.

Du fait que l'épaisseur de la kératoprothèse entre les deux jupes est sensiblement égale à l'épaisseur de la cornée, cela facilite la bio-colonisation de la jupe antérieure de la prothèse.

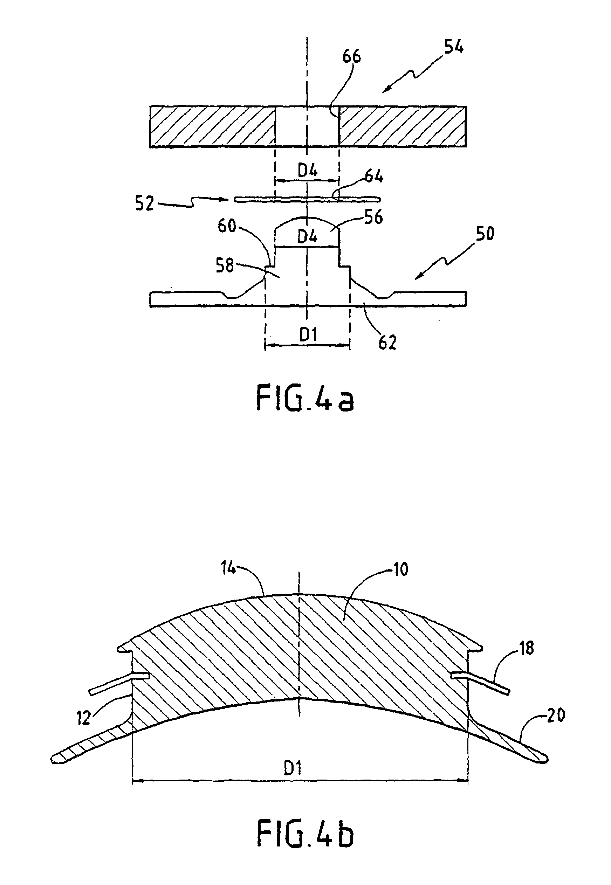

En se référant maintenant aux figures 4a et 4b, on va décrire un mode préféré mais non exclusif de fabrication de la kératoprothèse objet de l'invention.

Sur la figure 4a, on a représenté les trois pièces de départ qui sont une ébauche 50 de kératoprothèse, un disque ou pastille 52 réalisé avec le matériau bio-colonisable et une bague 54, l'ébauche 50 et la bague 54 étant réalisées avec le même matériau qui est par exemple du PMMA. L'ébauche 50 comprend une partie centrale 56 comportant une première partie cylindrique de diamètre D4 inférieur au diamètre D1 de la partie optique de la kératoprothèse à réaliser raccordée à une deuxième portion sensiblement cylindrique 58 par un épaulement 60. Le diamètre de cette deuxième partie est égal à D1. L'ébauche comprend enfin une partie formant une plaque 62 dans laquelle on taillera la jupe postérieure. La pastille 52 est percée d'un trou axial 64 de diamètre D4. Il en va de même de la bague 54 qui comporte l'orifice axial 66 de diamètre D4.

On place la pastille 52 sur l'épaulement 58 puis la bague 54 sur la pastille 52, la partie cylindrique 56 de l'ébauche étant engagée dans les trous axiaux de ces deux pièces.

Puis, par apport calorifique, par ultra-sons par exemple, on réalise le sondage de la bague 54 sur la partie cylindrique 56 de l'ébauche 50. La pastille 52 en matériau bio-colonisable est alors emprisonnée entre l'épaulement 58 et la bague 54.

Enfin, on réalise l'usinage de l'ébauche 50 et de la bague 54 pour donner la forme finale de la partie optique de la kératoprothèse et de sa jupe postérieure. La figure 4b montre la kératoprothèse obtenue par l'usinage convenable de l'ébauche et de la bague.

## Revendications

1. Dispositif de kératoprothèse pour être mis en place dans un orifice réalisé dans la cornée qui présente une épaisseur et une paroi interne, traversant le dispositif, comprenant une partie optique (10) en un premier matériau transparent présentant une face antérieure (14) définissant un dioptre optique, une paroi latérale (12) et une extrémité postérieure (16) ledit dispositif comprenant en outre une jupe antérieure annulaire (18) et une jupe postérieure annulaire (20),
ledit dispositif comprenant au total deux jupes constituées par ladite jupe antérieur (18) et ladite jupe postérieure (20);
ladite partie optique (10) comportant une paroi latérale (12) sensiblement cylindrique; ladite jupe antérieure (18) faisant saillie hors de la paroi latérale (12) de la partie optique (10) à proximité de sa face antérieure (14);
ladite jupe postérieure (20) faisant saillie hors de la paroi latérale (12) de la partie optique (10) à proximité de son extrémité postérieure (16);
la distance entre la face postérieure de la jupe antérieure (18) et la face antérieure de la jupe postérieure (20) étant sensiblement égale à l'épaisseur de la cornée par quoi la cornée est apte à être enserrée entre les deux jupes, ladite jupe postérieure (20) est apte à être appliquée contre la face interne de la cornée;
ledit dispositif **se caractérisant en ce que** ladite jupe antérieure (18) est réalisée en un polymère de synthèse microporeux bio-compatible et bio-colonisable distinct dudit premier matériau et peut être colonisée; ladite jupe postérieure (20) est réalisée avec ledit premier matériau pour avoir une résistance mécanique suffisante pour maintenir la partie optique (10) dans l'orifice ménagé dans la cornée.

2. Dispositif de kératoprothèse selon la revendication 1, **caractérisé en ce que** la face antérieure de la partie optique (10) définit un rebord qui recouvre partiellement la face antérieure de la jupe antérieure (20).

3. Dispositif de kératoprothèse selon l'une quelconque des revendications 1 et 2, **caractérisé en ce que** ladite partie optique (10) comporte un évidement dont le fond définit un deuxième dioptre optique.

4. Dispositif de kératoprothèse selon l'une quelconque des revendications 1 à 3, **caractérisé en ce que** ladite partie optique (10) comporte au moins un canal (30) de faible section dont une première extrémité débouche dans la face postérieure (16) de la partie optique (10) et dont la deuxième extrémité débouche dans sa paroi latérale (12) à proximité de la face postérieure de ladite jupe antérieure (18).

5. Dispositif de kératoprothèse selon l'une quelconque des revendications 1 à 4, **caractérisé en ce que** ladite jupe postérieure (20) est une pièce distincte de la partie optique (10), ladite jupe postérieure (20) étant vissée sur l'extrémité postérieure de la partie optique (10).

6. Dispositif de kératoprothèse selon l'une quelconque des revendications 1 à 5, **caractérisé en ce que** ladite partie optique (10) est réalisée en acrylique.

7. Dispositif de kératoprothèse selon la revendication 6, **caractérisé en ce que** la partie optique (10) est réalisée en PMMA.

8. Dispositif de kératoprothèse selon l'une quelconque des revendications 1 à 5, **caractérisé en ce que** ladite partie optique (10) est réalisée en un matériau souple choisi dans le groupe comprenant les gels de silicone et les acryliques.

9. Dispositif de kératoprothèse selon l'une quelconque des revendications 1 à 8, **caractérisé en ce que** ledit matériau bio-colonisable présente des pores dont le diamètre est compris entre 20 et 100 micromètres et représentant au moins 50% du volume total en vide.

10. Dispositif de kératoprothèse selon l'une quelconque des revendications 1 à 8, **caractérisé en ce que** ledit matériau biocolonisable est choisi parmi les matériaux utilisés pour la chirurgie réparatrice.

11. Dispositif de kératoprothèse selon la revendication 9, **caractérisé en ce qu'**il est réalisé en polytétrafluoroéthylène ou en polyéthylène.

12. Dispositif de kératoprothèse selon l'une quelconque des revendications 1 à 10, **caractérisé en ce que** le diamètre (D3) externe de la jupe antérieure (18) est compris entre 5 et 11 mm.

13. Dispositif de kératoprothèse selon l'une quelconque des revendications 1 à 12, **caractérisé en ce que** l'épaisseur de la jupe antérieure (18) est comprise entre 20 et 500 microns.

14. Dispositif de kératoprothèse selon l'une quelconque des revendications 1 à 13, **caractérisé en ce que** le diamètre (D1) de la partie optique (10) est compris entre 3 et 7 mm.

15. Dispositif de kératoprothèse selon l'une quelconque des revendications 1 à 14, **caractérisé en ce que** lesdites jupes antérieure (18) et postérieure (20) ont sensiblement parallèles entre elles.

## Patentansprüche

1. Keratoprothese-Einrichtung zum Einsetzen in eine in der Hornhaut ausgebildete Öffnung, die eine Dicke und eine innere Wand hat, die die Einrichtung überquert, mit einem optischen Bereich (10) aus einem ersten transparenten Material mit einer vorderen Fläche (14), die einen optischen Diopter definiert, einer Seitenwand (12) und einem hinteren Ende (16), welche Einrichtung außerdem eine vordere ringförmige Schürze (18) und eine hintere ringförmige Schürze (20) aufweist,
wobei die Einrichtung insgesamt zwei Schürzen aufweist, nämlich die vordere (18) und die hintere Schürze (20),
wobei der optische Bereich (10) eine im Wesentlichen zylindrische Seitenwand (12) aufweist,
wobei die vordere Schürze (18) von der Seitenwand (12) des optischen Bereichs (10) in der Nähe ihrer vorderen Fläche (14) hervorsteht,
wobei die hintere Schürze (20) von der seitlichen Wand (12) des optischen Bereichs (10) in der Nähe seines hinteren Endes (16) hervorsteht, und
wobei der Abstand zwischen der hinteren Fläche der vorderen Schürze (18) und der vorderen Fläche der hinteren Schürze (20) im Wesentlichen gleich der Dicke der Hornhaut ist, wodurch die Hornhaut zwischen den beiden Schürzen eingeschlossen werden kann, wobei die hintere Schürze (20) gegen die innere Fläche der Hornhaut anlegbar ist,
**dadurch gekennzeichnet, dass** die vordere Schürze (18) aus einem biokompatiblen und biokolonisierbaren mikroporösen Synthesepolymer besteht, das anders ist als das erste Material, und kolonisierbar ist; und dass die hintere Schürze (20) aus dem ersten Material ausgebildet ist, so dass sie einen ausreichenden mechanischen Widerstand hat, um den optischen Bereich (10) in der in der Hornhaut ausgebildeten Öffnung zu halten.

2. Keratoprothese-Einrichtung nach Anspruch 1, **dadurch gekennzeichnet, dass** die vordere Fläche des optischen Bereichs (10) einen Rand definiert, der teilweise die vordere Fläche der vorderen Schürze (18) bedeckt.

3. Keratoprothese-Einrichtung nach einem der Ansprüche 1 und 2, **dadurch gekennzeichnet, dass** der optische Bereich (10) eine Ausnehmung aufweist, deren Boden einen zweiten optischen Diopter definiert.

4. Keratoprothese-Einrichtung nach einem der Ansprüche 1 bis 3, **dadurch gekennzeichnet, dass** der optische Bereich (10) zumindest einen Kanal (30) mit geringem Querschnitt aufweist, dessen eines Ende in die hintere Fläche (16) des optischen Bereichs (10) mündet und dessen zweites Ende in die seitliche Wand (12) in der Nähe der hinteren Fläche der vorderen Schürze (18) mündet.

5. Keratoprothese-Einrichtung nach einem der Ansprüche 1 bis 4, **dadurch gekennzeichnet, dass** die hintere Schürze (20) ein von dem optischen Bereich (10) getrennt ausgebildetes Element ist, wobei die hintere Schürze (20) auf das hintere Ende des optischen Bereichs (10) aufgeschraubt ist.

6. Keratoprothese-Einrichtung nach einem der Ansprüche 1 bis 5, **dadurch gekennzeichnet, dass** der optische Bereich (10) aus Acryl besteht.

7. Keratoprothese-Einrichtung nach der Anspruch 6, **dadurch gekennzeichnet, dass** der optische Bereich (10) aus PMMA besteht.

8. Keratoprothese-Einrichtung nach einem der Ansprüche 1 bis 5, **dadurch gekennzeichnet, dass** der optische Bereich (10) aus einem weichen Material besteht, das aus der Gruppe ausgewählt ist, die Silikongels und Acryle aufweist.

9. Keratoprothese-Einrichtung nach einem der Ansprüche 1 bis 8, **dadurch gekennzeichnet, dass** das biokolonisierbare Material Poren hat, deren Durchmesser zwischen 20 und 100 Mikrometern liegt, wobei diese Poren zumindest 50% des gesamten Volumens als Leerräume ausmachen.

10. Keratoprothese-Einrichtung nach einem der Ansprüche 1 bis 8, **dadurch gekennzeichnet, dass** das biokolonisierbare Material aus den Materialien ausgewählt ist, die für die wiederherstellende Chirurgie verwendet werden.

11. Keratoprothese-Einrichtung nach der Anspruch 9, **dadurch gekennzeichnet, dass** die Einrichtung aus Polytetrafluorethylen oder Polyethylen besteht.

12. Keratoprothese-Einrichtung nach einem der Ansprüche 1 bis 10, **dadurch gekennzeichnet, dass** der Außendurchmesser (D3) der vorderen Schürze (18) zwischen 5 und 11 mm liegt.

13. Keratoprothese-Einrichtung nach einem der Ansprüche 1 bis 12, **dadurch gekennzeichnet, dass** die Dicke der vorderen Schürze (18) zwischen 20 und 500 Mikrometern liegt.

14. Keratoprothese-Einrichtung nach einem der Ansprüche 1 bis 13, **dadurch gekennzeichnet, dass** der Durchmesser (D1) des optischen Bereichs (10) zwischen 3 und 7 mm beträgt.

15. Keratoprothese-Einrichtung nach einem der Ansprüche 1 bis 14, **dadurch gekennzeichnet, dass** die vordere (18) und die hintere Schürze (20) im Wesentlichen parallel zueinander sind.

## Claims

1. A corneal prosthesis device for being put into place in an orifice made in the cornea which has a thickness and an inside wall that passes through the device, the device comprising an optical part (10) made of a first transparent material, the part having an anterior face (14) defining an optical surface, a side wall (12) and a posterior end (16), said device further comprising an annular anterior skirt (18) and an annular posterior skirt (20), said device comprising in all two skirts consisting of said anterior skirt (18) and said posterior skirt (20); said optical part (10) comprising a substantially cylindrical side wall (12); said anterior skirt (18) projecting from the side wall (12) of the optical part (10) in the vicinity of its anterior face (14), said posterior skirt (20) projecting from the side wall (12) of the optical part (10) in the vicinity of its posterior end (16), the distance between the posterior face of the anterior skirt (18) and the anterior face of the posterior skirt (20) being substantially equal to the thickness of the cornea whereby the cornea is adapted to be clamped between both skirts, said posterior skirt (20) is adapted to be pressed against the inside face of the cornea, said device being **characterised in that** said anterior skirt (18) is made of a biocompatible and biocolonizable microporous synthetic polymer distinct from said first material, and said skirt may be colonized; said posterior skirt (20) is made with said first material so as to provide sufficient mechanical strength to maintain the optical part (10) in the orifice provided in the cornea.

2. A corneal prosthesis device according to claim 1, **characterised in that** the anterior face of the optical part (10) defines a rim which partially overlies the anterior face of the anterior skirt (20).

3. A corneal prosthesis device according to claim 1 or 2, **characterised in that** said optical part (10) includes a recess whose end wall defines a second optical surface.

4. A corneal prosthesis device according to any one of claims 1 to 3, **characterised in that** said optical part (10) includes at least one small section channel (30) having a first end opening out into the posterior face (16) of the optical part (10) and having its second end opening out in the side wall (12) thereof close to the posterior face of said anterior skirt (18).

5. A corneal prosthesis according to any one of claims 1 to 4, **characterised in that** said posterior skirt (20) is a part that is distinct from the optical part (10), said posterior skirt (20) being screwed to the posterior end of the optical part (10).

6. A corneal prosthesis device according to any one of claims 1 to 5, **characterised in that** said optical part (10) is made of acrylic.

7. A corneal prosthesis device according to claim 6, **characterised in that** said optical part (10) is made of PMMA.

8. A corneal prosthesis device according to any one of claims 1 to 5, **characterised in that** said optical part (10) is made of a flexible material selected from the group comprising silicone gels and acrylics.

9. A comeal prosthesis device according to any one of claims 1 to 8, **characterised in that** said biocolonizable material has pores of a diameter lying in the range 20 µm to 100 µm, with the empty volume representing at least 50% of the total volume.

10. A corneal prosthesis device according to any one of claims 1 to 8, **characterised in that** said biocolonizable material is selected from the materials used in reconstructive surgery.

11. A corneal prosthesis device according to claim 9, **characterised in that** it is made of polytetrafluoroethylene or of polyethylene.

12. A corneal prosthesis device according to any one of claims 1 to 10, **characterised in that** the outside diameter (D3) of the anterior skirt (18) lies in the range 5 mm to 11 mm.

13. A corneal prosthesis device according to any one of claims 1 to 12, **characterised in that** the thickness of the anterior skirt (18) lies in the range 20 µm to 500 µm.

14. A corneal prosthesis device according to any one of claims 1 to 13, **characterised in that** the diameter (D1) of the optical part (10) lies in the range 3 mm to 7 mm.

15. A corneal prosthesis device according to any one of clams 1 to 14, **characterised in that** said anterior and posterior skirts (18, 20) are substantially parallel to each other.
